(19) Europäisches Patentamt
European Patent Office
Office européen des brevets

(11) **EP 4 519 013 B1**

(12) **EUROPEAN PATENT SPECIFICATION**

(45) Date of publication and mention
of the grant of the patent:
**15.04.2026  Bulletin 2026/16**

(21) Application number: **23723664.1**

(22) Date of filing: **20.04.2023**

(51) International Patent Classification (IPC):
*B01J 23/28* (2006.01)      *B01J 37/03* (2006.01)
*C07C 11/04* (2006.01)      *C07C 51/215* (2006.01)
*C07C 5/48* (2006.01)

(52) Cooperative Patent Classification (CPC):
(C-Sets available)
**B01J 23/28; B01J 37/03; C07C 5/48;**
C07C 2523/28; Y02P 20/52            (Cont.)

(86) International application number:
**PCT/IB2023/054055**

(87) International publication number:
**WO 2023/214236 (09.11.2023 Gazette 2023/45)**

(54) **MAKING CATALYSTS FOR OXIDATIVE DEHYDROGENATION**

HERSTELLUNG VON KATALYSATOREN ZUR OXIDATIVEN DEHYDRIERUNG

FABRICATION DE CATALYSEURS DESTINÉS À LA DÉSHYDROGÉNATION OXYDATIVE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **02.05.2022  US 202263337403 P**

(43) Date of publication of application:
**12.03.2025  Bulletin 2025/11**

(73) Proprietor: **Nova Chemicals (International) S.A.
1700 Fribourg (CH)**

(72) Inventors:
• **SULLIVAN, David
Calgary, Alberta T2E 1Z9 (CA)**
• **SIMANZHENKOV, Vasily
Calgary, Alberta T3G 5C3 (CA)**

(74) Representative: **Mewburn Ellis LLP
Aurora Building
Counterslip
Bristol BS1 6BX (GB)**

(56) References cited:
**WO-A1-2006/130288      WO-A1-2022/167967**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 5/48, C07C 11/04**

**Description**

TECHNICAL FIELD

The present disclosure relates generally to catalysts and systems for oxidative dehydrogenation (ODH). More specifically, the catalyst contains molybdenum (Mo), vanadium (V), tellurium (Te), tantalum (Ta), and oxygen (O).

BACKGROUND ART

Olefins like ethylene, propylene, and butylene, are basic building blocks for a variety of commercially valuable polymers. Since naturally occurring sources of olefins do not exist in commercial quantities, polymer producers rely on methods for converting the more abundant lower alkanes into olefins. The method of choice for today's commercial scale producers is steam cracking, a highly endothermic process where steam-diluted alkanes are subjected very briefly to a temperature of at least 800°C. The fuel demand to produce the required temperatures and the need for equipment that can withstand that temperature add significantly to the overall cost. In addition, the high temperature promotes the formation of coke, which accumulates within the system, resulting in the need for costly periodic reactor shutdowns for maintenance and coke removal.

Selective oxidation processes, such as oxidative dehydrogenation (ODH), are an alternative to steam cracking that are exothermic and produce little or no coke. In ODH, a lower alkane, such as ethane, is mixed with oxygen in the presence of a catalyst and optionally an inert diluent, such as carbon dioxide or nitrogen or steam, which may be performed at temperatures as low as 300°C, to produce the corresponding alkene. Various other oxidation products may be produced in this process, including carbon dioxide and acetic acid, among others. ODH suffers from lower conversion rates when compared to steam cracking, a fact that when combined with lower selectivity may have prevented ODH from achieving widespread commercial implementation.

There is a need for a catalyst for an ODH of ethane process with high ethylene selectivity. It has been demonstrated that the $MoVNbTeO_x$ catalyst used in ODH processes exhibits permanent activity and selectivity loss over time at elevated ODH temperatures. The robustness of the catalyst to oxygen-depleted ODH conditions has also been tested and it has been found that though the catalyst activity would recover completely after air regeneration cycles, the selectivity would only partially recover, falling below 90% at 25% conversion. It was shown that running the reactor at a low temperature of 350°C with low space velocity caused a smaller loss in selectivity of the catalyst as compared to subjecting the catalyst to temperatures above 360°C. Therefore, applying the $MoVNbTeO_x$ catalyst in a commercial ODH process would require the plant to operate at both lower temperature and GHSV (gas hourly space velocity) in order to maintain high catalyst performance. These process restrictions would require a larger reactor volume size resulting in higher costs, a narrower temperature operating window, and ultimately more difficult operation of the reactor. WO 2006/130288 discloses a method for selectively oxidizing ethane to ethylene.

SUMMARY OF INVENTION

An embodiment described in examples herein provides a method for preparing a catalyst. The method includes forming a slurry including oxides of molybdenum, tantalum oxide, and tellurium and adding $VOSO_4$ to the slurry. Citric acid, oxalic acid, and ethylene glycol are added to the slurry. The slurry is transferred to an autoclave, and the autoclave is heated to form a catalyst precursor. The catalyst precursor formed in the autoclave is isolated and calcined to form the catalyst.

In an aspect, anhydrous tantalum oxide is used as the tantalum oxide in the slurry. In an aspect, the tantalum oxide is prepared using $TaCl_5$ by hydrolyzing $TaCl_5$ in ammonium hydroxide to prepare $Ta_2O_5 \cdot 3H_2O$.

In an aspect, the autoclave is heated at 190°C for 48 hours.

In an aspect, the catalyst precursor is calcined by placing the catalyst precursor in a furnace under a purified nitrogen atmosphere, ramping a temperature of the furnace from ambient to a temperature of 600°C over 6 hours, and holding the temperature at 600°C for 2 hours.

In an aspect, the catalyst includes the formula: $Mo_aV_bTe_cTa_dO_x$, wherein a is 1.0, b is about 0.01 to about 0.3, c is about 0.01 to about 0.09, d is about 0.01 to about 0.05, and x is the number of oxygen atoms necessary to render the catalyst electronically neutral.

In an aspect, b is about 0.2 to about 0.4, c is about 0.03 to about 0.07, and d is about 0.03 to about 0.05.

In an aspect, the catalyst has the formula: $Mo_1V_{0.3}Te_{0.05}Ta_{0.05}O_x$.

In an aspect, the catalyst has a temperature of 25% ethane conversion of about 367°C and a selectivity at 25% ethane conversion of about 95%.

Another embodiment described in examples provides a process for oxidative dehydrogenation of ethane, the process including contacting a gaseous feed including ethane and oxygen with a catalyst in a reactor to produce an effluent including ethylene, wherein the catalyst has the formula: $Mo_aV_bTe_cTa_dO_x$. In this formula, a is 1.0, b is about 0.01 to about 0.3, c is about 0.01 to about 0.09, d is about 0.01 to about 0.05, and x is the number of oxygen atoms necessary to render the catalyst electronically neutral. The catalyst is prepared by forming a slurry including oxides of molybdenum, tantalum oxide, and tellurium and adding VOSO4 to the slurry. Citric acid, oxalic acid, and ethylene glycol are added to the slurry. The slurry is transferred to an autoclave, and the autoclave is heated to form a catalyst precursor. The catalyst precursor formed in the autoclave is isolated and calcined to form the catalyst.

In an aspect, b is about 0.2 to about 0.4, c is about 0.03 to about 0.07, and d is about 0.03 to about 0.05. In an aspect, the catalyst has the formula: $Mo_1V_{0.3}Te_{0.05}Ta_{0.05}O_x$.

In an aspect, the process includes preparing the catalyst using anhydrous $Ta_2O_5$ without further preparation as a precursor.

In an aspect, the process includes preparing the catalyst using $TaCl_5$ by hydrolyzing $TaCl_5$ in ammonium hydroxide to prepare $Ta_2O_5 \cdot 3H_2O$, and using the $Ta_2O_5 \cdot 3H_2O$ to prepare the catalyst.

In an aspect, the catalyst has a temperature of 25% ethane conversion of about 367°C and a selectivity at 25% ethane conversion of about 95%.

BRIEF DESCRIPTION OF THE DRAWINGS

Figure 1 is an autoclave used for preparing catalysts.

Figures 2A and 2B show a test apparatus for testing the ODH performance of the catalysts.

Figure 3 is a plot of the conversion of reagents to ethylene for comparative catalyst 2C before calcination.

Figure 4 is a plot of the selectivity of ethylene formation for comparative catalyst 2C before calcination.

Figure 5 is a plot of the conversion of reagents to ethylene for comparative catalyst 2C.

Figure 6 is a plot of the selectivity of ethylene formation for comparative catalyst 2C.

Figure 7 is a plot of the conversion of reagents to ethylene for sample 3E before calcination.

Figure 8 is a plot of the selectivity of ethylene formation for sample 3E before calcination.

Figure 9 is a plot of the conversion of reagents to ethylene for sample 3E after calcination in nitrogen.

Figure 10 is a plot of the selectivity of ethylene formation for sample 3E after calcination in nitrogen.

Figure 11 is a plot of the conversion/space yields for sample 3E.

Figure 12 is a plot of the conversion of reagents to ethylene for sample 4E.

Figure 13 is a plot of the selectivity of ethylene formation for experiment a catalyst 4E.

Figure 14 is a plot of the conversion/space yields for sample 4E.

Figure 15 is a plot of the conversion of reagents to ethylene for sample 5E.

Figure 16 is a plot of the selectivity of ethylene formation for sample 5E.

Figure 17 is a plot of the conversion of reagents to ethylene for sample 6E.

Figure 18 is a plot of the selectivity of ethylene formation for sample 6E.

Figure 19 is a plot of the conversion/space yields for sample 6E.

Figure 20 is a plot of the conversion of ethylene formation for sample 7E.

Figure 21 is a plot of the selectivity of ethylene formation for sample 7E.

Figure 22 is a plot of the conversion of ethylene formation for sample 8E.

Figure 23 is a plot of the selectivity of ethylene formation for sample 8E.

Figure 24 is a plot of the conversion of ethylene formation for sample 9E.

Figure 25 is a plot of the selectivity of ethylene formation for sample 9E.

DESCRIPTION OF EMBODIMENTS

Selective oxidation (SO) is generally used in oxidative dehydrogenation (ODH) reactions to form ethylene, or other alpha-olefins, from ethane. Embodiments described herein provide a synthesis method for a MoVTaTe catalyst, for example, for the ODH process. In the method, catalyst precursor powders are mixed, for example, by being ground together, and then used in a hydrothermal synthesis process to form the catalyst materials. Previous procedures used a tantalum (V) chloride ($TaCl_5$) precursor to form a hydrated tantalum (V) oxide ($Ta_2O_5$), which was used as the precursor to form the catalyst as testing had shown that anhydrous tantalum (V) oxide required HF or other strong or toxic acids for digestion to form the hydrated tantalum (V) oxide.

However, the digestion procedure adds significant costs. As described herein, it has been determined that a commercial, anhydrous tantalum (V) oxide could be used directly in the catalyst synthesis process without further processing.

In addition to a simpler procedure, in embodiments of the catalysts described herein, lower amounts of tantalum (Ta) and tellurium (Te) than in other MoVTaTe catalysts were used, reducing the costs. For example, the catalyst shows increased activity and higher stability than hydrothermally generated catalysts.

In some embodiments, the $MoVTeTaO_x$ catalyst is stable at operation above 400°C, maintaining high activity and selectivity. In some embodiments, the catalyst maintains high activity and selectivity after a slight initial activity loss. In some embodiments, the slight initial activity loss is attributed to catalyst equilibration. The enhanced stability is beneficial because it provides the option to operate at higher reactor temperatures than previously considered. Higher temperature are generally associated with higher conversion but at the cost of reduced selectivity and potential deactivation over time of the catalyst. Catalyst described in various embodiments operate at higher temperatures with higher conversion while maintaining selectivity and without activity loss over time.

In some embodiments, the $MoVTeTaO_x$ catalyst exhibits high tolerance to low residual oxygen conditions while producing high conversion and selectivity. This is beneficial because it should allow for more conversion and in turn higher ethylene yields without negative impact to the catalyst life.

In some embodiments, anhydrous tantalum oxide is digested into aqueous solution by either using hydrofluoric acid or oxalic acid. For the safer aqueous oxalic acid digestion to succeed in dissolving tantalum, highly hydrated tantalum oxalate must be used, which is costly and may be difficult to source and obtain. The use of anhydrous tantalum oxalate, for example, used directly in the catalyst synthesis alleviates this obstacle, as it is more readily available and easier to store.

Another important benefit of using commercial anhydrous tantalum pentoxide precursor is the fact that this material has a long shelf life and simpler storage requirements, while the hydrated tantalum oxide hydroxide has rather complicated storage conditions to avoid dehydration to avoid spoilage. Hydrated tantalum oxide would require very tight environment specification for storage, which would further complicate the scale up of the synthetic method.

Provided herein is an oxidative dehydrogenation catalyst material that includes molybdenum (Mo), vanadium (V), tellurium (Te), tantalum (Ta), and oxygen (O). The catalyst is represented by the formula MoVTeTaOx. In some embodiments, the catalyst has the formula $Mo_aV_bTe_cTa_dO_x$. In some embodiments, the catalyst has the formula $Mo_1V_{0.01-0.3}Te_{0.01-0.9}Ta_{0.01-0.05}$. In some embodiments, the catalyst has the formula $Mo_1V_{0.39-0.49}Te_{0.1-0.3}Ta_{0.01-0.05}$. In some embodiments, the catalyst has the formula $Mo_1V_{0.3}Te_{0.05}Ta_{0.05}$. In each of these formulations, oxygen is present in sufficient amounts to render the catalyst electrically neutral.

In some embodiments, a is 1.0.

In some embodiments, b is about 0.01 to about 0.3. In some embodiments, b is about 0.1 to about 0.3. In some embodiments, b is about 0.3.

In some embodiments, c is about 0.01 to about 0.09. In some embodiments, c is about 0.01 to about 0.07. In some embodiments, c is about 0.05.

In some embodiments, d is about 0.01 to about 0.05. In some embodiments, d is about 0.03 to about 0.05. In some embodiments, d is about 0.05.

In some embodiments, x is the number of oxygen atoms necessary to render the catalyst electronically neutral.

Also provided herein is a catalyst material that includes a catalyst, such as a catalyst of the present disclosure, and a catalyst support or carrier. Some supports are particularly suitable for the catalyst, for example, they are chemically compatible (there is no substantial impact on ethylene selectivity). Other supports may be less compatible, meaning they may lead to substantial reduction of catalyst performance, for example, ethylene selectivity. Consequently, not just any support can be chosen; the support should be selected in a judicious matter based off both short-term and longer-term catalysis performance testing. In some embodiments, there is an emphasis on long-term testing showing no loss of selectivity with time on stream (for example, TOS of >48 hours). In some embodiments, the catalyst support or carrier is selected from the group consisting of precipitated synthetic silica, fumed synthetic silica, silica-alumina, $\alpha$-alumina, and anatase titania. In some embodiments, the catalyst support or carrier is precipitated synthetic silica.

As used herein, the term "catalyst material" refers to a material that includes an active catalyst that can promote the oxidative dehydrogenation of ethane to ethylene, for example, on a support. The catalyst material can be a plurality of particles or a formed catalyst material. Non-limiting examples of formed catalyst materials include extruded catalyst materials, pressed catalyst materials, and cast catalyst materials. Non-limiting examples of pressed and cast catalyst materials includes pellets-such as tablets, ovals, and spherical particles.

As used herein, the term "catalyst" generally refers to the active catalyst portion of a catalyst material. The catalyst is generally processed in further steps to form a catalyst material. The catalyst material may also be processed in further steps to form a final catalyst material.

Conversion of the ethane feed gas was calculated as a volume flow rate change of ethane in the product compared to feed ethane mass flow rate using the following formula:

$$C = \left( \frac{2 * X_{Ethylene} + X_{CO2} + X_{CO}}{2 * X_{Ethylene} + 2 * X_{Ethane} + X_{CO2} + X_{CO}} \right) * 100\%$$

In the above equation, C is the percent of ethane feed gas that has been converted from ethane to another product (i.e., ethane conversion) and X is the molar concentration of the corresponding compound in the gaseous effluent exiting the reactor at corresponding temperature. The ethane conversion was then plotted as a function of temperature to acquire a linear algebraic equation. The linear equation for ethane conversion was solved to determine the temperatures in which the ethane conversion of 35% and 50% were achieved (i.e., 35 and 50% conversion temperatures).

Furthermore, the gas exiting the reactor was analyzed by gas chromatography to determine catalyst or catalyst material selectivity to ethylene (i.e., the percentage on a molar basis of ethane that forms ethylene). Selectivity to ethylene was determined using the following equation:

$$S_{Ethylene} = \left( \frac{2 * X_{Ethylene}}{2 * X_{Ethylene} + X_{CO2} + X_{CO}} \right) * 100\%$$

In the above equation, $S_{Ethylene}$ is the selectivity to ethylene and X is the molar concentration of the corresponding compound in the gaseous effluent exiting the reactor at corresponding temperature. After the 35 and 50% conversion temperature was determined, the above equation for selectivity was solved using the corresponding values for $X_{Ethylene}$, $X_{CO2}$, and $X_{CO}$ at the 35% conversion temperature.

GHSV (gas hourly space velocity) is defined as volumetric flow of the reactor feed gas divided by the volume of the catalyst bed. For GHSV values of Catalyst Materials, the catalyst bed is treated as catalyst only (not including support) where an assumption was made that the total volume of the catalyst material measured when multiplied by the wt.% of catalyst is the volume of the catalyst. The GHSV was calculated based off the measured volume of the pressed particles (before mixing with quartz sand) and varied depending on each catalyst or catalyst material bulk density. For catalyst materials, the GHSV reported is for the catalyst only, where an assumption was made that the total volume of the catalyst material measured when multiplied by the wt.% of catalyst is the volume of the catalyst.

As used herein, the term "oxidative dehydrogenation" or "ODH" refers to processes that couple the endothermic dehydrogenation of an alkane with the strongly exothermic oxidation of hydrogen as is further described herein. For testing catalysts, the ODH reactions herein are assumed to be referring to the ODH of ethane (ODE).

Other than in the operating examples or where otherwise indicated, all numbers or expressions referring to quantities of ingredients, reaction conditions, etc. used in the specification and claims are to be understood as modified in all instances by the term "about". Accordingly, unless indicated to the contrary, the numerical parameters set forth in the following specification and attached claims are approximations that can vary depending upon the desired properties, which the present disclosure desires to obtain. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the disclosure are approximations, the numerical values set forth in the specific examples are reported as precisely as possible. Any numerical values, however, inherently contain certain errors necessarily resulting from the standard deviation found in their respective testing measurements.

In addition, it should be understood that any numerical range recited herein is intended to include all sub-ranges subsumed therein. For example, a range of "1 to 10" is intended to include all sub-ranges between and including the recited minimum value of 1 and the recited maximum value of 10; that is, having a minimum value equal to or greater than 1 and a maximum value of equal to or less than 10. Because the disclosed numerical ranges are continuous, they include every value between the minimum and maximum values. Unless expressly indicated otherwise, the various numerical ranges specified in this application are approximations.

## EXAMPLES

### Reagents

Reagents purchased from manufacturers were used as received, without further purification. The following chemicals were purchased from Sigma Aldrich: Ammonium molybdate ($(NH_4)_6Mo_7O_{24} \cdot 4H_2O$; Product No. A7302), vanadium(IV) oxide sulfate hydrate ($VOSO_4 \cdot 3.41H_2O$; Product No. 233706), telluric acid ($Te(OH)_6$; Product No. 86375), oxalic acid ($C_2H_2O_4$; Product No. 75688), ammonium metavanadate ($NH_4VO_{3(s)}$; Product No. 398128), 95-98% sulfuric acid ($H_2SO_4$; Product No. 258105), fumed synthetic amorphous silica (Product No. S5130), aluminum silicate (Product No. 343358), anatase titania (Product No. 232033), and zirconium(IV) oxide (Product No. 204994).

The vendor supplied certificates of analysis were used to establish the hydrate content for different batches of reagents (e.g. vanadium (IV) oxide sulfate hydrate). Ammonium hydroxide, 28-30 wt.%, was purchased from Anachemia, VWR Company under the Product No. 05566-461.

$TaCl_5$ (CAS 7721-01-9) was purchased from Acros organics (99.9%) or Plazmotherm (99.99%). The commercial anhydrous $Ta_2O_5$ (CAS 1314-61-0, 99.8 % $Ta_2O_5$) was used as purchased from Plazmotherm.

The following chemicals were purchased from Alfa Aesar: colloidal zirconia (Cat No. 40124; 20 wt.% $ZrO_2$ dispersed in water) and colloidal alumina (Cat No. 12733; 20 wt.% $Al_2O_3$ dispersed in water). The following chemical were acquired from Saint-Gobain: $\alpha$-alumina (DENSTONE® 99) and silicon carbide (product code SC55167, 13 wt.% $SiO_2$ and the balance SiC). The precipitated synthetic amorphous silica was obtained from PQ Corporation, product identifier CS6846; PD-10042. The boehmite aluminum oxide hydroxide was obtained from Honeywell UOP, product name VERSAL® V-250 (No. 86251). The calcium titanate was purchased from Goodfellow Corporation, product code CA546, which is classified as 80-100 wt.% calcium titanium oxide ($CaTiO_3$).

### Preparation of Catalyst Materials

### Catalyst Precursors

The $Nb_2O_5 \cdot 3H_2O$ was prepared from $NbCl_5$ (Sigma-Aldrich, 98%, CAS 10026-12-7) by dissolving the $NbCl_5$ in an excess of an $NH_4OH$ aqueous solution. The hydrated oxide sample was X-ray amorphous. The amount of water was determined by TGA analysis. Specifically, a weighed portion of niobium chloride was suspended in an aqueous solution of ammonia (approximately 25%) by slowly adding $NbCl_5$ to stirred aqueous solution in the molar ratio of $Nb/NH_4OH$ of not more than 1/3.

### Hydrothermal Catalyst Preparation

### Preparation of Sample 1C

Weighed 11.37 g of $Nb_2O_5 \cdot H_2O$ and approximately 127 mL of de-ionized water into a 250 mL RBF. Added 19.09 g of oxalic acid ($H_2C_2O_4$) and stirred at 65°C for 24 hours resulting in a turbid white solution of niobium oxalate.

Weighed 84.28 g of $(NH_4)_6Mo_7O_{24} \cdot 4H_2O$ and approximately 200 mL of de-ionized water into a 500 mL round bottom flask (RBF), stirred to dissolve the powder. Weighed 18.27 g of telluric acid into a 100 mL beaker and approximately 50 mL of de-ionized water, the mixture was stirred at approximately 50°C to dissolve the powder. The telluric acid solution was added to the $(NH_4)_6Mo_7O_{24} \cdot 4H_2O$ solution dropwise at room temperature. Heated the Mo/Te solution to approximately 80°C using an oil bath. 28.0 mL of NH4OH solution was added dropwise to the Mo/Te solution to adjust the pH to 7.5 at 80°C; the contents of the flask was left to stir at 80°C for 2 hours. 28.0 mL of 5 M $H_2SO_4$ solution was added dropwise to the flask to adjust the pH to 4.99. The contents of the flask was stirred at 80°C for approximately 2 hours.

Weighed 70.04 g of $VOSO_4 \cdot 3.41H_2O$ and approximately 150 mL of de-ionized water into a 250 mL beaker; the mixture was stirred to dissolve the powder. In a 2L RBF, the $VOSO_4$ solution was added dropwise using a dropping funnel to the Mo/Te solution at 70°C and stirred for approximately 30 minutes, forming an opaque purple solution.

The previously prepared niobium oxalate solution was added slowly into the purple solution using a dropping funnel at 70°C and stirred for approximately 30 minutes, forming a greenish slurry with a purple film on a walls of the RBF. The green slurry was transferred to a 2L glass liner with a large magnetic stir bar.

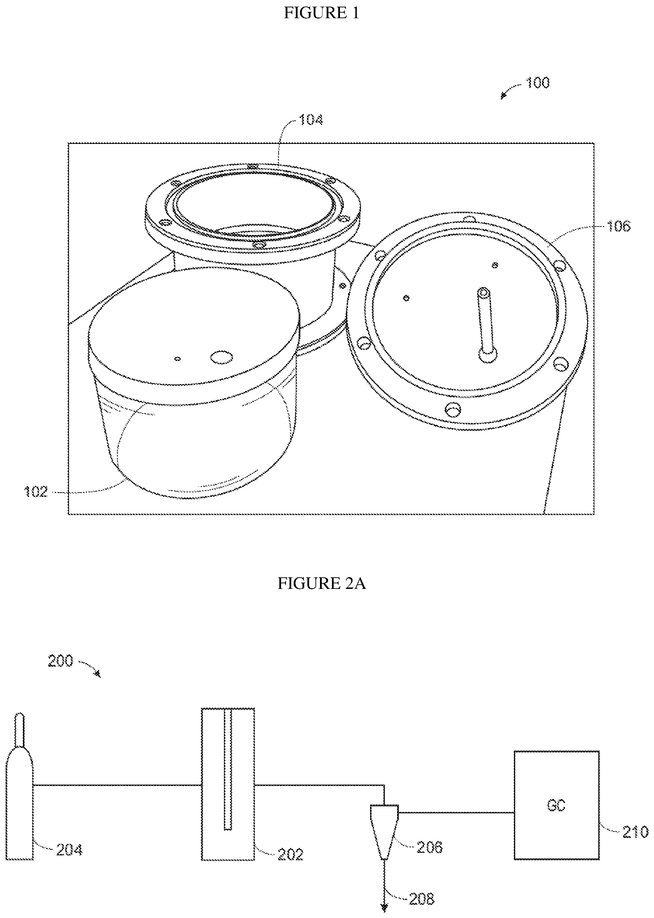

Figure 1 is an autoclave 100 used for preparing catalysts. The autoclave 100 is a 2 L PARR reactor, available from Parr Instrument Company of Moline, IL, USA.

A glass liner 102 holds the sample slurry. The glass liner 102 is placed inside the steel body 104 of the autoclave 100 and the cover 106 is sealed to the steel body 104. In some examples, the autoclave 100 is slowly filled with nitrogen and evacuated 10 times to remove air in the headspace. The autoclave 100 is left overnight (about 10 to about 16 hours) at about 15 psig nitrogen and stirring at about 200 rpm. The nitrogen headspace pressure was released through a bubbler to purge the air out of the lines.

After stirring overnight, a backpressure regulator on the autoclave 100 was set to 160 psig, the temperature controller was raised to 170°C and a condenser water bath on an outline line was set to 25°C. The temperature profile was monitored. The reaction was left to stir at 200 rpm for an additional 17 hours at 165°C, for a total of about 24 hours.

The heat was turned off, top valve closed, insulation removed and the reactor was allowed to cool overnight while stirring at 200 rpm. The pressure in the PARR reactor was released by opening the top valve in the fume hood.

The PARR reactor was disassembled and the glass liner was removed. The contents were filtered using an aspirator through a Buchner funnel with four Whatman #4 filter papers (available from Sigma Aldrich). The resulting filter cake was washed with de-ionized water until the filtrate was clear. The filter cake was placed in an oven at 90°C to dry over a weekend (about 48 to about 60 hours) and allowed to cool to room temperature.

A portion of the dried filter cake was manually ground using a mortar and pestle. It was observed that the catalyst was hard and brittle. The ground catalyst was loaded the catalyst into boats and calcined at 600°C under a nitrogen flow.

Preparation of Sample 2C

Sample 2C, $MoV_{0.30}Te_{0.05}Nb_{0.05}O_x$, was also prepared using the hydrothermal method. In this procedure, 5.3964 g of $MoO_3$ (Sigma-Aldrich, >99.5%, CAS 1313-27-5), 1.0236 g of $V_2O_5$, (Sigma-Aldrich, ≥98.0%, CAS 1314-62-1), 0.3008 g of $TeO_2$, (Acros organics, 99+%, CAS 7446-07-3), 0.3014 g of $Nb_2O_5 \cdot 3H_2O^*$, 0.5413 g of citric acid (Sigma-Aldrich, 99%, CAS 77-92-9), 0.1690 g of oxalic acid, anhydrous (Fluka analytical > 99%, CAS 144-62-7) and 0.1766 g of ethylene glycol (Aldrich, 99.8% anhydrous, CAS 107-21-1) were dispersed simultaneously in 200 mL of distilled water at ambient temperature.

The obtained slurry was transferred in a stainless-steel autoclave with a glass liner (Figure 1). The autoclave was immediately sealed without purging the remaining air volume with inert gas. The contents of the autoclave were mixed at a speed of 500 rpm using a magnetic stirrer. Stirring of the mixture started simultaneously with the temperature ramp. Syntheses was performed at 190°C for 48 hours. The temperature in the autoclave was controlled and maintained using a thermo-controller. Once the temperature program was completed, the reacting slurry in the vessel was cooled down to room temperature while keeping on stirring. The solid is separated by filtration, washed with 2.5 times the volume of distilled water and dried in static air at 80°C. The mass of the catalyst after drying was 4.7253 g.

Preparation Details for Experimental Examples MoV(Te)TaO Mixed Oxide Catalysts

A series of synthesis conditions were tested to compare the preparations and formulations of the catalysts. The conditions that were adjusted for each sample are shown in Table 1.

General Procedures for Making Catalysts Shown in Table 1

Catalyst Precursors

The hydrated $Ta_2O_5 \cdot 3H_2O$, mentioned in footnote 1, was prepared by dissolving $TaCl_5$ (Acros organics, 99.9%, CAS 7721-01-9) in an excess of an $NH_4OH$ aqueous solution. The hydrated oxide sample was X-ray amorphous. The water amount was determined by TGA analysis.

The commercial anhydrous $Ta_2O_5$ (CAS 1314-61-0, 99.8% $Ta_2O_5$) was used as purchased from Plazomotherm. According to the TGA analysis, this sample is thermoamorphous.

Table 1: Conditions Adjusted for Each Sample are Highlighted in the Table in Bold.

| Sample[1] | MoO$_3$ (g) | V$_2$O$_5$ (g) | VOSO$_4$ (g) | TeO$_2$ (g) | Ta$_2$O$_5$ (g) | Nb$_2$O$_5$ (g) | CA (g) | OA (g) | EG (g) | Sample Weight (g)[3] | Catalyst Formula |
|---|---|---|---|---|---|---|---|---|---|---|---|
| 1C | | SEE PROCEDURE ABOVE | | | | | | | | | MoV$_{0.3}$Te$_{0.17}$Nb$_{0.13}$O$_x$ |
| 2C | 5.3964 | 1.0236 | | 0.3008 | | 0.3014 | 0.5413 | 0.1690 | 0.1766 | 4.7253 | MoV$_{0.30}$Te$_{0.05}$Nb$_{0.05}$O$_x$ (partially hydrolyzed NbCl$_5$) |
| 3E | 5.3989 | 1.0264 | | 0.2994 | 0.4651[2] | | 0.5460 | 0.1706 | 0.1788 | | MoV$_{0.30}$Te$_{0.05}$Te$_{0.05}$O$_x$ (partially hydrolyzed TaCl$_5$) |
| 4E | 5.4010 | 1.0266 | | 0.3010 | 0.4670 | | 0.5432 | 0.1719 | 0.1831 | | MoV$_{0.30}$Te$_{0.05}$Ta$_{0.05}$O$_x$ (Fresh TaCl$_5$) |
| 5E[4] | 5.3984 | 1.0261 | | 0.2991 | 0.4654[2] | | 0.5405 | 0.1693 | 0.1842 | 4.8786 | MoV$_{0.30}$Te$_{0.05}$Ta$_{0.05}$O$_x$ (partially hydrolyzed TaCl$_5$) |
| 6E | 5.3951 | 1.0257 | | 0.2985 | 0.4189[3] | | 0.5437 | 0.1680 | 0.1726 | 5.6554 | MoV$_{0.30}$Te$_{0.05}$Ta$_{0.05}$O$_x$ |
| 7E[5] | 5.3967 | 1.0269 | | 0.1509 [5] | 0.4669[2] | | 0.5407 | 0.1706 | 0.2072 | 5.2020 | MoV$_{0.30}$Te$_{0.025}$Ta$_{0.05}$O$_x$ |
| 8E[6] | 5.4002 | 1.0259 | | 2.2984 | 0.2351[2] | | 0.5437 | 0.1718 | 0.1833 | 5.1030 | MoV$_{0.30}$Te$_{0.05}$Ta$_{0.025}$O$_x$ |
| 9E[7] | 5.3985 | | 3.0705 | 0.3059 | 0.4662[2] | | 0.5456 | 0.1705 | 0.1762 | 5.6033 | MoV$_{0.30}$Te$_{0.05}$Ta$_{0.025}$O$_x$ |

[1] C indicates comparative samples, E indicates experimental samples
[2] Hydrated Ta$_2$O$_5$ prepared from TaCl$_5$
[3] Commercial anhydrous Ta$_2$O$_5$ as purchased
[4] Synthesis performed for 24 hours, versus 48 hours for sample 3E
[5] Test at ½ tellurium
[6] Test at ½ tantalum
[7] Test run using VOSO$_4$ as the vanadium source

Preparation of Sample 3E ($MoV_{0.30}Te_{0.05}Ta_{0.05}O_x$, Catalyst Prepared From Partially Hydrolyzed $TaCl_5$)

In the first step, 5.3989 g of $MoO_3$, 1.0264 g of $V_2O_5$, 0.2994 g of $TeO_2$, 0.4651 g of $Ta_2O_5 \cdot 3H_2O^*$, 0.5460 g of citric acid, 0.1706 g of oxalic acid, and 0.1788 g of ethylene glycol were dispersed simultaneously in 200 mL of distilled water at ambient temperature. The obtained slurry was transferred in a stainless-steel autoclave with a glass liner. The autoclave was immediately sealed without purging the remaining air volume with inert gas. Stirring of the mixture started simultaneously with the temperature ramp. Synthesis was performed at 190°C for 48 hours. Once the temperature program was finished, the reacting slurry in the vessel was cooled down to room temperature while keeping on stirring. The solid was separated by filtration, washed with 2.5 times the volume of distilled water and dried in static air at 80°C.

The $Ta_2O_5 \cdot 3H2O$ was prepared by dissolving $TaCl_5$ (Acros organics, 99.9%, CAS 7721-01-9) in an excess of an $NH_4OH$ aqueous solution. The hydrated oxide sample was X-ray amorphous. The amount of water was determined by TGA analysis. Specifically, a weighed portion of tantalum chloride was suspended in an aqueous solution of ammonia (approximately 25%) by slowly adding $TaCl_5$ to stirred aqueous solution in the molar ratio of Ta to $NH_4OH$ of not more than 1 to 3. In this example, a partial hydrolysis of the starting chloride had occurred during storage, potentially interfering with complete dissolution. The suspensions were stirred on a magnetic stirrer at room temperature for 3 hours, then stirring was stopped and precipitation was left to form for a day. After a day, the precipitates were filtered and washed repeatedly with distilled water until a negative reaction to chloride ion (with silver nitrate) was obtained. Next, the precipitates were dried at room temperature for a day.

According to the XRD data, the sample was crystalline, despite the fact that it was not calcined. Calcination only leads to minor changes in the XRD. As the tantalum chloride had partially hydrolyzed during storage, the experiment was repeated using a fresh sample of tantalum chloride.

Preparation of Sample 4E ($MoV_{0.30}Te_{0.05}Ta_{0.05}O_x$, Catalyst Prepared From Fresh $TaCl_5$).

$Ta_2O_5 \cdot 3H_2O$ was obtained by dissolving of fresh $TaCl_5$ (Plasmotherm, 99.99%) in excess of $NH_4OH$ aqueous solution. This was performed in a glass beaker (volume 500 mL), containing 150 mL of 1M ammonia solution, 10 g (27.9 mmol, 139.6 mmol Cl) of tantalum chloride was added. As the reaction was very exothermic and released gas (HCl), the reaction was performed slowly by adding small portions with of the $TaCl_5$ while vigorously stirring.

After adding the entire amount of tantalum chloride, and after the exothermic reaction was complete, 100 mL of distilled water was added to the beaker. The contents of the glass were stirred using a magnetic stirrer for 2 hours, then left to settle overnight. The resulting tantalum oxide was repeatedly washed with distilled water by decantation until a neutral pH was reached. The precipitate was then filtered on a Buchner funnel through a paper filter and washed again with distilled water until there was no reaction with silver nitrate. The resulting white powder was dried in air at room temperature for 24 hours. The hydrated oxide sample was X-ray amorphous.

5.4010 g $MoO_3$, 1.0266 g $V_2O_5$, 0.3010 g $TeO_2$, 0.4670 g $Ta_2O_5 \cdot 3H_2O$, 0.5432 g citric acid, 0.1719 g oxalic acid, and 0.1831 g ethylene glycol were dispersed simultaneously in 200 mL of distilled water at ambient temperature to form a slurry. The slurry was transferred to a stainless steel autoclave with a glass liner (Figure 1). The autoclave was sealed without purging the remaining air volume with inert gas. The stirring of the mixture started simultaneously with temperature ramp. Syntheses was performed at 190°C for 48 hours. Once the temperature program has finished, the reacting slurry in the vessel is cooled down to room temperature while maintaining stirring. The solid is separated by filtration, washed with 2.5 times the volume of distilled water and dried in static air at 80°C.

The dried catalyst precursor was grinded into a using a mortar/pestle. Catalyst precursor material was loaded in a quartz boat and the boat was placed into glass furnace tube, which is used for calcinations. The setup was purge under purified nitrogen flow (< 0.3 ppm $O_2$) and then the heating program on the furnace was started (RT to 600°C in 6 hours, held at 600°C for 2 hours). The calcination proceeded under a slow stream (30 ml/min) of purified nitrogen (vent through silicone oil bubbler). The solids obtained were a dark grey powder, which were ground, mold into tablets, ground again, and then sieved to collected fraction 0.8-1.0 mm resulting in a final catalyst, which were tested for activity and selectivity.

General Catalyst Synthesis

The synthesis of the remaining experimental examples, shown in Table 1, were also performed hydrothermally. The Mo-, V-, Te-. Ta-containing precursors and citric acid (CA), oxalic acid (OA), and ethylene glycol (EG), in the amounts described above, were dispersed in 200 mL of distilled water at ambient temperature. The commercial tantalum oxide used for sample 6E was dispersed in the slurry without further treatment. For each sample, the slurry was transferred to a stainless-steel autoclave with a glass liner (Figure 1). The autoclave was immediately sealed without purging the remaining air volume with inert gas. The stirring of the mixture was started simultaneously with the temperature ramp. The syntheses were performed at 190°C for 48 hours (unless otherwise specified). Once the temperature program was finished, the reacting slurry in the vessel was cooled down to room temperature while maintaining stirring. The solid was separated by filtration, washed with 2.5 times the volume of distilled water, and dried in static air at 80°C overnight.

The commercial, anhydrous $Ta_2O_5$ was directly used in place of the $Ta_2O_5 \cdot H_2O$ in the preparation of sample 6E. Previous tests indicated that this would not be functional without digestion of the anhydrous $Ta_2O_5$ using very strong acids, such as HF, as anhydrous tantalum oxide is particularly difficult to dissolve into aqueous mixtures (compared to the other oxides). However, as described herein, the substitution provided excellent results, possibly due to the lower amount in the catalyst than in previous studies. Accordingly, the substitution provided a much lower cost material than previous precursors, such as $TaCl_5$, or $Ta(OEt)_5$ used in other studies.

After synthesis, the catalyst materials were ground into a powder using a mortar/pestle. The catalyst materials were loaded in a quartz boat, and the boat was placed into a quartz furnace tube, which was used for the calcination. The setup was purged with a purified nitrogen flow (< 0.3 ppm $O_2$), and then a heating program on the furnace was started, with a ramp rate from ambient temperature to 600°C in 6 hours, and then the furnace was held at 600°C for 2 hours. The calcination proceeded under a slow stream of purified nitrogen, about 30 ml/min, that was vented through a silicone oil bubbler to prevent air infiltration. The solids obtained were ground, pressed into tablets and ground again, and then sieved to collect a fraction of 0.8-1.0 mm resulting in a final catalysts, which were tested for activity and selectivity.

Instruments and Measurements

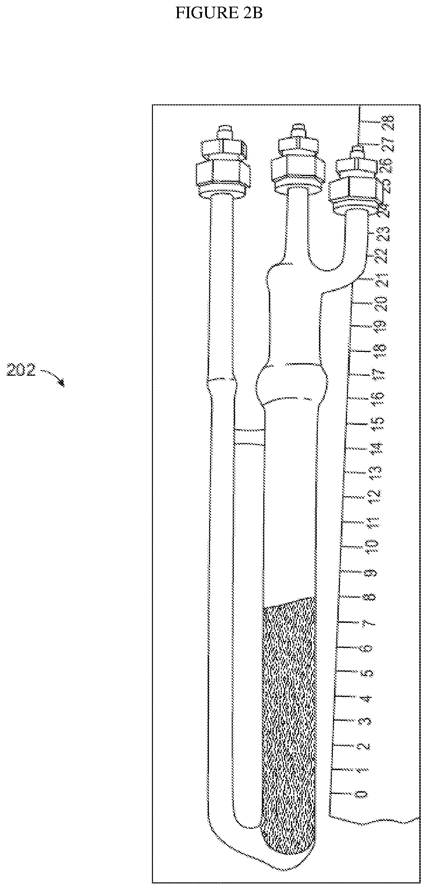

Figures 2A and 2B show a test apparatus for testing the ODH performance of the catalysts. The catalysts were prepared for testing via pressing the calcined catalyst powder into the shape of rectangular prism, using a press force of 11 metric tons. The pressed material was crushed to produce finer particles, and sieved to have the catalyst particles fraction of 0.25-0.75 mm, which was tested as a representative catalyst sample via procedure described below.

Figure 2A is schematic drawing of test apparatus 200 for testing the ODH performance of the catalysts. The catalysts were tested in ethane oxidation at 320 - 410°C by placing the sample charge (0.1 cm$^3$, ~0.15 g, fraction 0.25-0.75 mm) in a fixed-bed quartz reactor 202. The weight of catalyst was about 0.13g.

A mixed gas feed cylinder 204 was coupled to the fixed-bed quartz reactor 202 through a stainless steel (SS316L) ethane/oxygen (77/23 volume ratio) supply line. The outlet of the fixed-bed quartz reactor 202 was connected to a stainless steel (SS316L) tubing routing the outgoing product gas flow passed through a glass water trap 206 (20°C) for removal of the excess water and any condensable materials 208. Samples of the reactor effluent were injected periodically into an online chromatograph 210 equipped with the Poropac-Q column and a TCD detector. The catalyst performance was measured with an accuracy $\pm 5\%$. The connection between the quartz tube and the stainless steel lines (1/16") was done, using Teflon ferrules. The fixed-bed quartz reactor 202 was wrapped with heating tape and placed in an insulation housing. The probe of a thermal couple was placed on the outside of the quartz tube wall where catalyst zone is located.

The flow rate of the ethane/oxygen gas mixture was adjusted to 600 cm$^3$/hour. The reactor was heated to 320°C with a flow of mixed ethane and oxygen and stabilized for 40 minutes. GC samples were taken periodically in 10°C to 20°C steps. For each measurement step, the reactor was stabilized for 40 minutes before injecting the sample to the online GC.

As shown in Figure 2B, the reactor 202 is a U shaped quartz tube (OD: 6 mm, ID: 4 mm) that was filled with a uniform mixture of equal volume (0.1 cm$^3$ each) of catalyst and quartz particles (0.75-1 mm sizes). The reactor volume that was not occupied by catalyst bed was filled with quartz particles (0.75-1 mm sizes).

The reaction mixtures used for testing was about 75 vol. % ethane and about 25 vol. % oxygen, although some variation around this number, such as 78 vol. % ethane and about 22 vol. % oxygen, do not substantially affect the results. The gas chromatograph 232 was a Chromatek-Kristall 5000 with a thermal conductivity detector, using a column type-M ss316 3M*2mm, Hayesep Q 80/100 mesh, and a column M ss316 3M*2mm, NaX 60/80 mesh. The column temperature was ramped according to the following program: 40°C for 1.5 minutes, then rising to 100°C at a speed of 15°C/min. For the corresponding calculations, the standard correction coefficients presented in Table 2 were used.

Table 2. Correction Coefficients

|  | Correction Coefficient |
|---|---|
| $C_2H_6$ | 0.59 |
| $C_2H_4$ | 0.585 |
| $CH_4$ | 0.45 |
| HCOOH | 0.65 |
| $CO_2$ | 0.915 |
| CO | 0.67 |

To accurately identify the resulting products, the exact retention time of each component of the mixture, including reagents and all possible products of this reaction, was determined using the gas chromatograph 232 before conducting the experiment. Table 3 shows the retention times of the mixture components.

Table 3. Retention Times of the Mixture Components for the First Part of the Experiment.

| Component | Retention Time, min |
|---|---|
| Air | 0.7 |
| $CO_2$ | 2.2 |
| $C_2H_6$ | 5.1 |
| $H_2O$ | 7.5 |
| $C_2H_4$ | 4.6 |
| HCOOH (>99,9%) | 14.8 |
| $CH_3COOH$ (>99,9%) | 19.5 |
| $CH_3COH$ (>99,9%) | 16.3 |
| $CH_3OH$ (>99,9%) | 16.2 |

Summary of Catalyst Performance

The results on the comparative samples for from partially hydrolyzed $TaCl_5$, fresh $TaCl_5$, and commercial anhydrous $Ta_2O_5$, are summarized in Tables 4 and 5.

Table 4. Temperatures of 25%-Conversion of Ethane ($T_{25\%}$) and Corresponding Values of Selectivity of Ethylene Formation (S) for Three Catalysts of the Formula $MoV_{0.3}Te_{0.05}Ta_{0.05}O_x$.

| Catalyst | Source of Ta | $T_{25\% \text{ ethane conv.}}$, °C | S, % |
|---|---|---|---|
| 3E | Precipitated oxide-hydroxide from partially hydrolyzed $TaCl_5$ | 350 | 96 |
| 4E | Precipitated oxide-hydroxide from fresh $TaCl_5$ | 368 | 95.5 |
| 6E | Commercial tantalum oxide (anhydrous $Ta_2O_5$) | 334 | 98.1 |

Table 5. Temperatures of 99$^+$%-conversion of $O_2$ ($T_{99+\%}$) and Corresponding Values of Selectivity of Ethylene Formation (S) for Three Catalysts of the Formula $Mo_1V_{0.3}Te_{0.05}Ta_{0.05}O_x$.

| Catalyst | Source of Ta | $T_{99\% \text{ O2 conv.}}$, °C | S, % |
|---|---|---|---|
| 3E | Precipitated oxide-hydroxide from partially hydrolyzed $TaCl_5$ | 392 | 93.5 |
| 4E | Precipitated oxide-hydroxide from fresh $TaCl_5$ | 397 | 93.2 |
| 6E | Commercial oxide | 365 | 97 |

As one can see, sample 4E, made with fresh $TaCl_5$, as compared to sample 3E, made with partially hydrolyzed $TaCl_5$, demonstrates slightly lower activity in the low-temperature region of low ethane conversion (Table 4). However, the activity/selectivity of the two samples is similar at higher conversions (Table 5). Accordingly, the source of, and storage of, the starting $TaCl_5$ used for oxide-hydroxide precipitation does not substantially affect quality of the final catalyst.

The third sample 6E, made using anhydrous tantalum oxide, exceeds both samples prepared from precipitated oxide-hydroxide in activity and selectivity of ODH, as shown in Tables 4 and 5. This is a surprising result as previous studies indicated that anhydrous tantalum oxide needed to be digested prior to use, for example, using HF or oxalic acid. In the formation of samples 6E, the anhydrous tantalum oxide was directly substituted for the precipitated tantalum oxide and provided a more effective catalyst.

Table 6. Activity and Selectivity of Some Catalysts Calcined in $N_2$ and Tested in Identical Conditions.

| Formula | Sample Number | Temperature of 25% Ethane Conversion, °C | Selectivity at 25% Conversion, % |
|---|---|---|---|
| $Mo_1V_{0.3}Te_{0.17}Nb_{0.13}O_x$ | 1C | 357 | 98 |
| $Mo_1V_{0.3}Te_{0.05}Nb_{0.05}O_x$ | 2C | 350 | 97.5 |
| $Mo_1V_{0.3}Te_{0.05}Ta_{0.05}O_x$ | 3E | 350 | 96 |
| $Mo_1V_{0.3}Te_{0.05}Ta_{0.05}O_x$ | 4E | 368 | 95.5 |
| $Mo_1V_{0.3}Te_{0.05}Ta_{0.05}O_x$ | 5E | 338 | 96.5 |
| $Mo_1V_{0.3}Te_{0.05}Ta_{0.05}O_x$ | 6E | 334 | 98.1 |
| $Mo_1V_{0.3}Te_{0.025}Ta_{0.05}O_x$ | 7E | 324 | 95.8 |
| $Mo_1V_{0.3}Te_{0.05}Ta_{0.0025}O_x$ | 8E | 325 | 96.5 |
| $Mo_1V_{0.3}Te_{0.05}Ta_{0.05}O_x$ | 9E | 367 | 95 |

Table 6 includes data for other samples for comparison. Sample 1C represents an earlier generation of ODH catalyst that contains higher levels of tellurium and niobium. Sample 1C is synthesized via a hydrothermal method using ionic precursors. Sample 2C was made using lower amounts of niobium to determine the efficacy.

Discuss of results for individual catalysts.

Comparative Example 2C (not calcined):

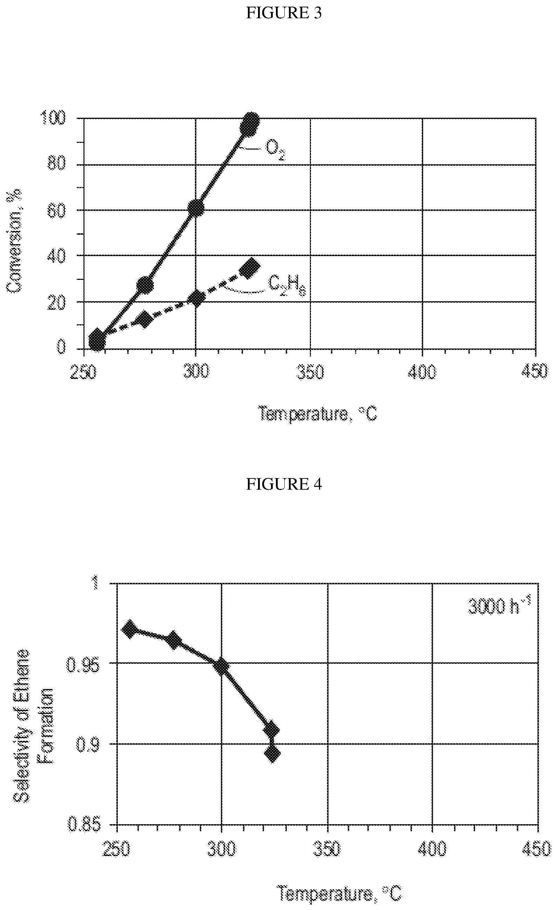

Figure 3 is a plot of the conversion of reagents to ethylene for comparative catalyst 2C (0.128 g) before calcination. Figure 4 is a plot of the selectivity of ethylene formation for comparative catalyst 2C before calcination.

The results obtained are quite impressive: the non-calcined catalyst demonstrates activity at an unusually low temperature. The complete conversion of oxygen is reached at ~330°C, with the selectivity to ethylene formation being on the level of 90%. Evaluation of the temperature of the 25% ethane conversion gives the value T25% = 308°C, with the selectivity ~92.5%. Accordingly, the catalysts with the formula $Mo_1V_{0.3}Te_{0.05}Nb_{0.05}O_x$ demonstrate a high activity combined with a reasonably high selectivity.

Sample 2C calcined:

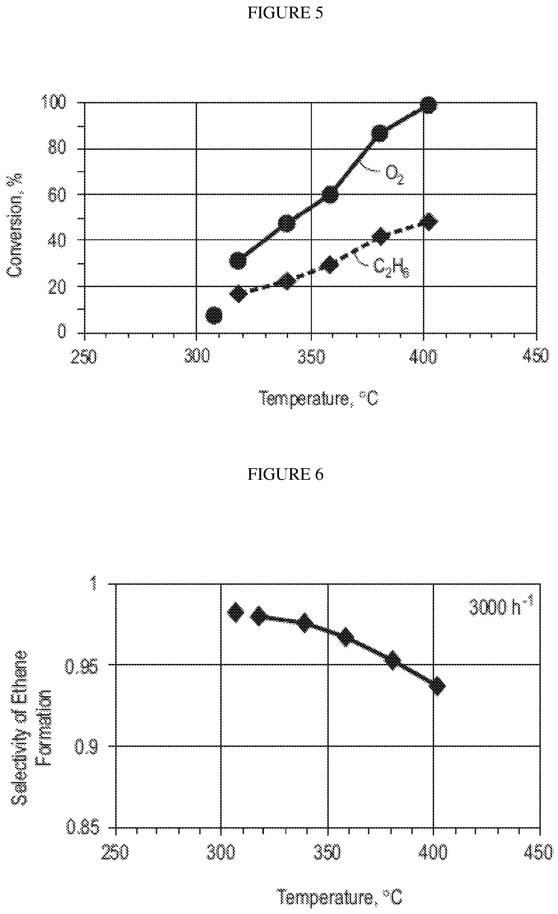

For fully formulated catalysts, the active phase is generally calcines, followed by a separate calcination of the fully formulated catalyst, to distinguish these two calcining steps. Therefore, the sample 2C was calcined at 600°C in pure $N_2$ and tested for comparison. Figure 5 is a plot of the conversion of reagents to ethylene for comparative catalyst 2C after calcination in pure nitrogen at 600°C. Figure 6 is a plot of the selectivity of ethylene formation for comparative catalyst 2C.

Comparison of the results obtained demonstrates a drastic loss of the catalytic activity for the calcined sample. For example. T25% for the sample 2C increases from 308°C to ~350°C due to calcination. On the other hand, the loss of activity is accompanied by a substantial overall increase of the selectivity. As a result, a positive effect is reached: the operation of the catalyst at a higher temperature provides a higher selectivity of ethylene formation (97% at T25% = 350°C). In other words, more effective and productive ODH can be reached at increased temperatures on the calcined sample).

It can be noted that both the activity and selectivity of the calcined sample 2C reach the level of the 4-component samples containing substantially larger amounts of Nb and Te.

Sample 3E:

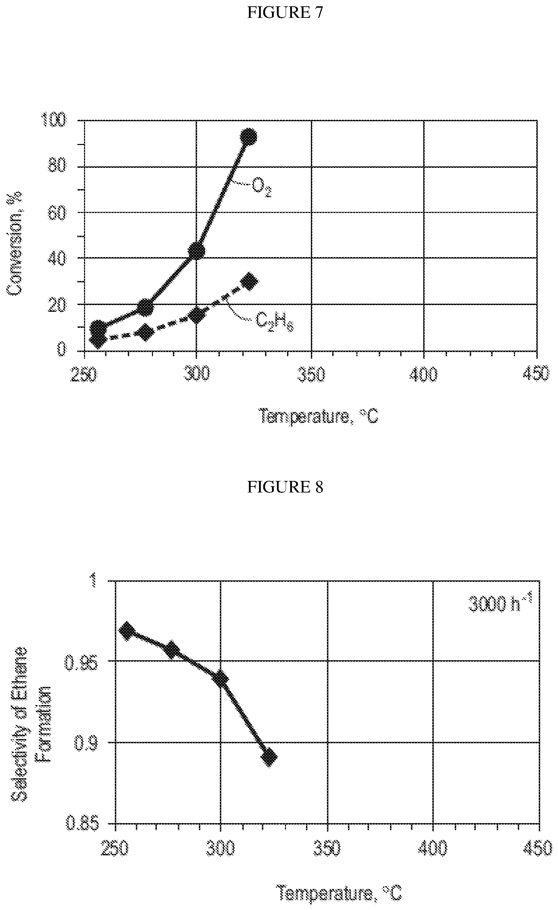

Figure 7 is a plot of the conversion of reagents to ethylene for sample 3E before calcination. Figure 8 is a plot of the selectivity of ethylene formation for sample 3E before calcination. The measurements were performed using 0.134 g of the catalyst under a gas mixture of 75 vol. % ethane and 25 vol. % oxygen at a flow rate of 600 $cm^3$ per hour.

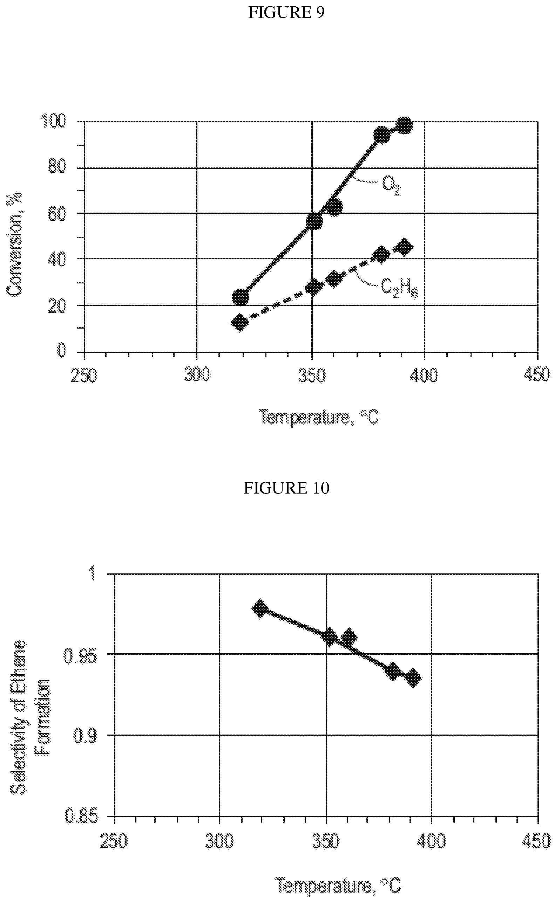

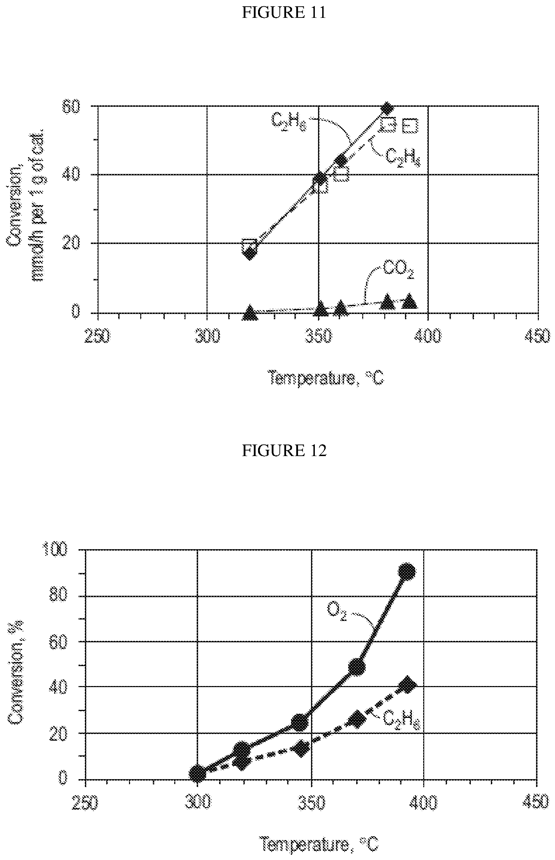

Figure 9 is a plot of the conversion of reagents to ethylene for sample 3E after calcination in nitrogen. Figure 10 is a plot of the selectivity of ethylene formation for sample 3E after calcination at 600°C under nitrogen. The measurements were performed using 0.134 g of catalyst under a gas mixture of 77 vol. % ethane and 23 vol. % oxygen at a flow rate of 600 $cm^3$ per hour and a GHSV = 3000 $h^{-1}$. Figure 11 is a plot of the conversion/space yields for sample 3E.

The results obtained for the tantalum catalyst coincide surprisingly well with data collected for the niobium. As for the niobium catalyst, the non-calcined sample demonstrates activity at an unusually low temperature, and the complete oxygen conversion is reached at T ~325°C. Once more, calcination of the sample in $N_2$ at 600°C causes a sharp loss of activity but this effect is compensated by a considerable increase of the selectivity (Figures 9-11).

This part of work is focused on repeated preparation and testing of four-component $Mo_1V_{0.3}Te_{0.05}Ta_{0.05}O_x$ sample 3E made hydrothermally by modified method and synthesized with use of pure stoichiometric $TaCl_5$. This sample is prepared according NOVA Chemicals' order for patent application started earlier with the sample 3E.

Sample 4E:

Catalytic testing of the four-component $Mo_1V_{0.3}Te_{0.05}Ta_{0.05}O_x$ catalyst, sample 4E, made with fresh $TaCl_5$ was performed under the same conditions as those used in testing sample 3E, e.g., 300 - 400°C at a gas flow rate 600 $cm^3 h^{-1}$ using a gas mixture of 75 vol. % ethane and 25 vol. % oxygen.

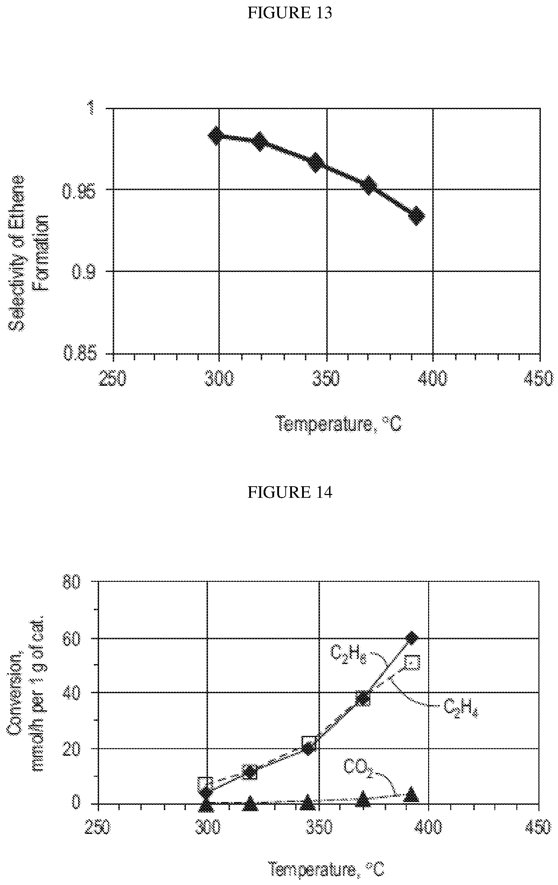

Figure 12 is a plot of the conversion of reagents to ethylene for sample 4E. Figure 13 is a plot of the selectivity of ethylene formation for experiment a catalyst 4E. The experiments were run using 0.13 g of catalyst with a gas mixture of 75 vol. % ethane and 25 vol. % oxygen at a flow rate of 600 $cm^3$ per hour with a GHSV = 3000 $h^{-1}$. Figure 14 is a plot of the conversion/space yields for sample 4E.

The data for samples 4E, 3E, and 6E are compared in Tables 4 and 5. The catalyst prepared using the fresh $TaCl_5$ was slightly less effective than the catalyst prepared with the partially hydrolyzed $TaCl_5$, possible due to morphological changes from the slower hydrolysis.

Sample 5E:

Variation of time of $Mo_1V_{0.3}Te_{0.05}Ta_{0.05}O_x$ hydrothermal synthesis.

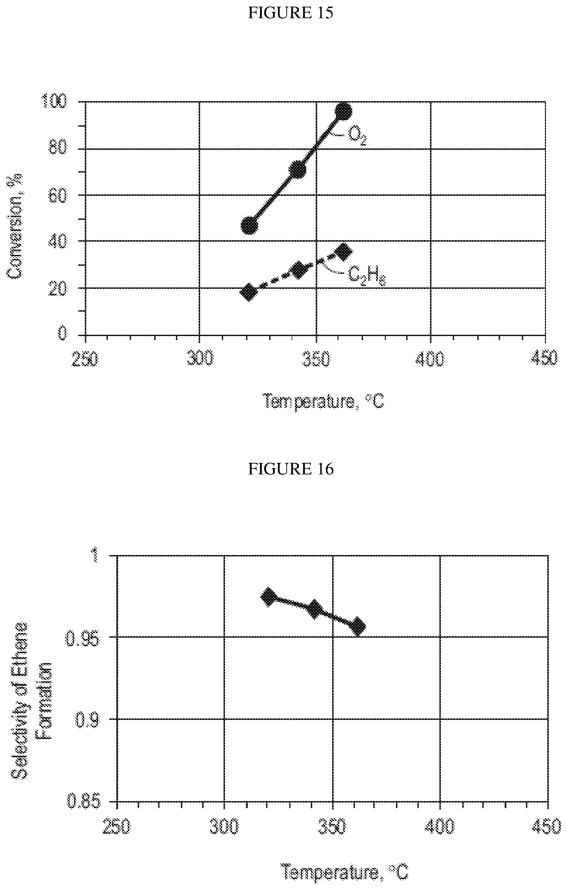

Figure 15 is a plot of the conversion of reagents to ethylene for sample 5E. Figure 16 is a plot of the selectivity of ethylene formation for sample 5E. The time for the hydrothermal synthesis of $Mo_1V_{0.3}Te_{0.05}Ta_{0.05}O_x$ was shortened from 48 hours to 24 hours for sample 5E. The sample 5E was calcined in standard conditions (pure $N_2$, 600°C, 2 h) and tested in an ODH reaction. A 0.139 g sample of catalyst was tested using a gas mixture of 78 vol. % Ethane and 22 vol. % Oxygen at a flow rate of 600 $cm^3$/h, and a GHSV = 3000 $h^{-1}$.

Surprisingly, sample 5E (run at 24 h) demonstrates an activity/selectivity pattern that slightly exceeds the same parameters measured for sample 3E (48 h) (shown in Figures 12 and 13). Thus, the time used for the hydrothermal synthesis can be shortened substantially without any loss of the $Mo_1V_{0.3}Te_{0.05}Ta_{0.05}O_x$ catalyst performance. However, the amount of sample 5E prepared by the 24-hour synthesis (~4.9 g) is measurably smaller than the amount of the sample 3E formed in the 48-hour hydrothermal synthesis (~5.6 g).

Sample 6E:

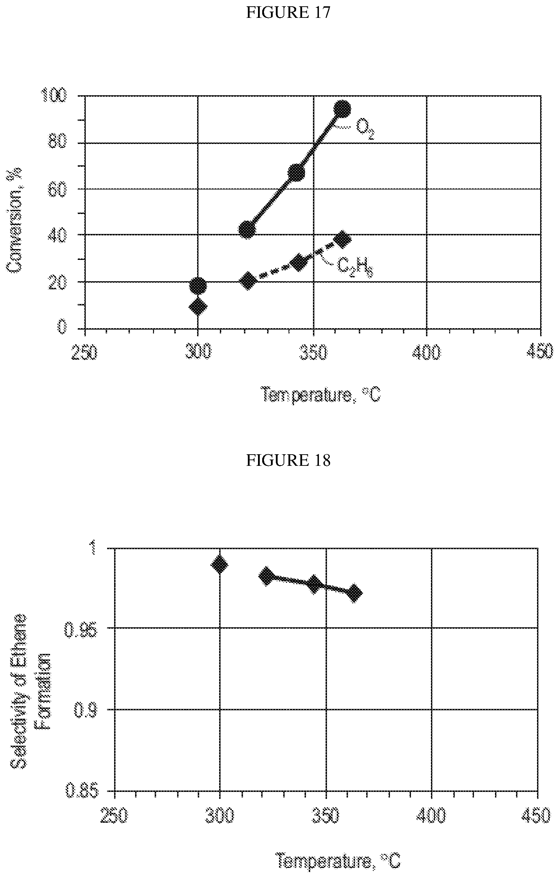

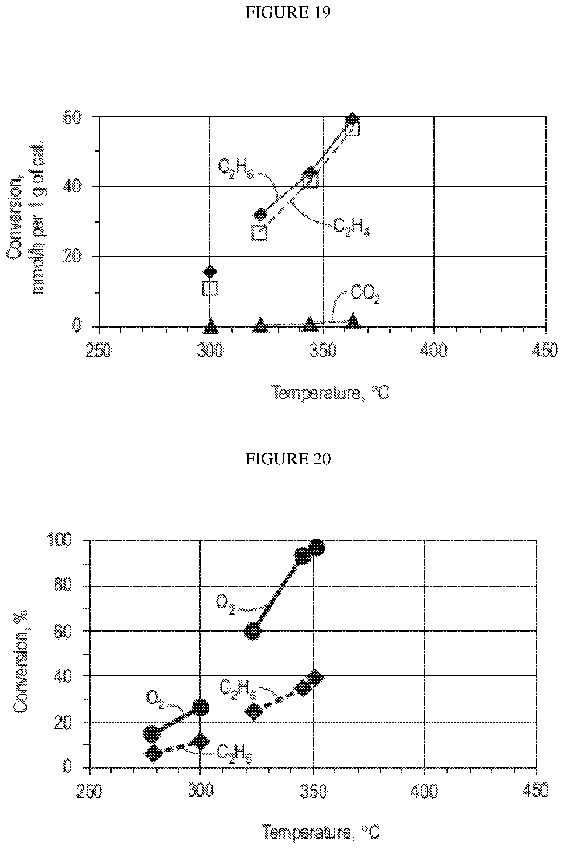

Figure 17 is a plot of the conversion of reagents to ethylene for sample 6E. Figure 18 is a plot of the selectivity of ethylene formation for sample 6E. Figure 19 is a plot of the conversion/space yields for sample 6E. The tests were run using 0.129 g of catalyst with a gas mixture of 78 vol. % ethane and 22 vol. % oxygen, at a flow rate of 600 $cm^3$/h and a GHSV = 3000 $h^{-1}$.

As shown in Tables 4 and 5, sample 6E, made from commercial, anhydrous tantalum oxide, had better performance than both samples 3E and 4E prepared from freshly precipitated oxide-hydroxide in both activity and selectivity of ODH of ethane. Further, as shown in Table 6, sample 6E showed better performance than either of the control samples 1C and 2C.

Comparison of temperatures at 25% ethane conversion and corresponding selectivity values demonstrates outstanding performance of all three samples containing low amounts of Ta and Te, prepared by hydrothermal synthesis. In addition, the use of commercial, anhydrous $Ta_2O_5$ in the synthesis provides the best result.

This conclusion is even better illustrated by comparison of the results obtained in the severe testing conditions of complete O2 conversion, shown in Table 5, which showed a 99+% conversion of oxygen. This high level of selectivity (S = 97%) was not achieved at the ~100% $O_2$ conversion with other catalysts.

Sample 7E:

In attempt to test the effects of further reductions in the metal content of the catalyst, the content of Te, was reduced by a factor of two to prepare sample 7E, $Mo_1V_{0.3}Te_{0.025}Ta_{0.05}O_x$. After the hydrothermal synthesis was completed, the samples was calcined in standard conditions, i.e., under pure $N_2$ at 600°C for 2 hours. The catalyst was tested in the ODH of ethane.

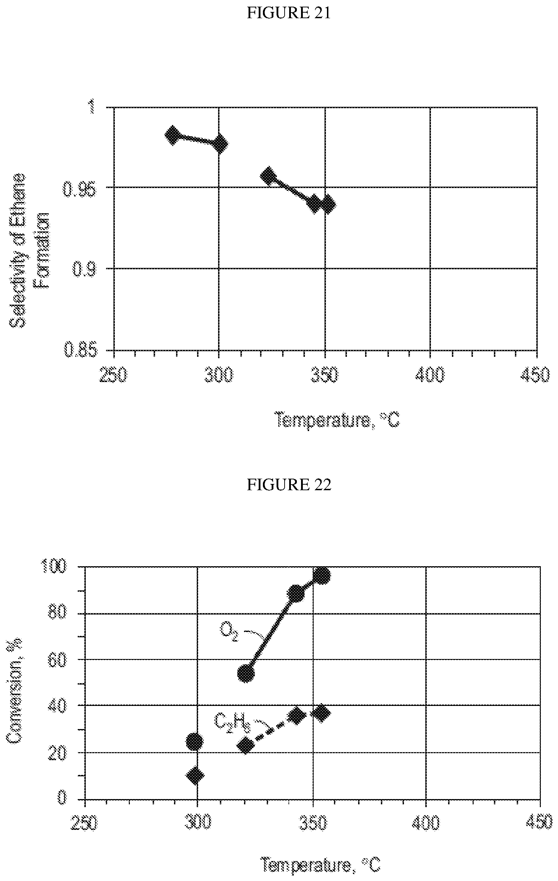

Figure 20 is a plot of the conversion of ethylene formation for sample 7E. Figure 21 is a plot of the selectivity of ethylene formation for sample 7E. The test was run on 0.129 g of catalyst using a gas mixture of 78 vol. % ethane and 22 vol. % oxygen at a flow rate of 600 $cm^3$/h and a GHSV = 3000 $h^{-1}$.

<u>Sample 8E:</u>

Another sample with reduced metal content, in this case tantalum content of 0.025, was prepared for comparison. After preparation by hydrothermal synthesis was completed, sample 8E ($Mo_1V_{0.3}Te_{0.05}Ta_{0.025}O_x$), was calcined in standard conditions, i.e., under pure N2 at 600°C for 2 hours. The catalyst was tested in the ODH of ethane.

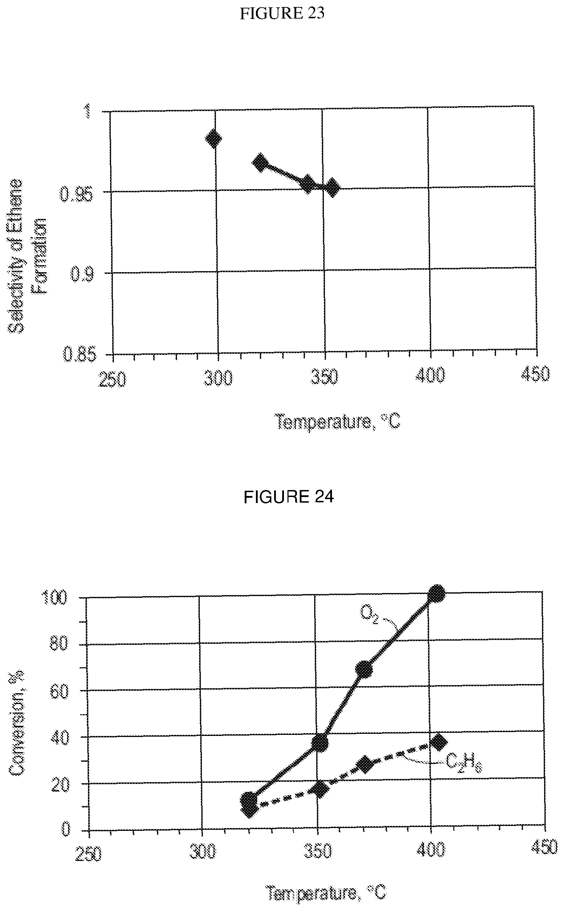

Figure 22 is a plot of the conversion of ethylene formation for sample 8E. Figure 23 is a plot of the selectivity of ethylene formation for sample 8E. The test was run on 0.129 g of catalyst using a gas mixture of 78 vol. % ethane and 22 vol. % oxygen at a flow rate of 600 $cm^3$/h and a GHSV = 3000 h-1.

The results for samples 7E and 8E are shown in Table 6. While both of the samples show a reduction in the operating temperature to achieve 25% conversion, neither showed as high a selectivity as the control samples 1C and 2C. Further, neither of the samples 7E or 8E match the selectivity of the sample 6E prepared using the anhydrous tantalum oxide.

<u>Sample 9E:</u>

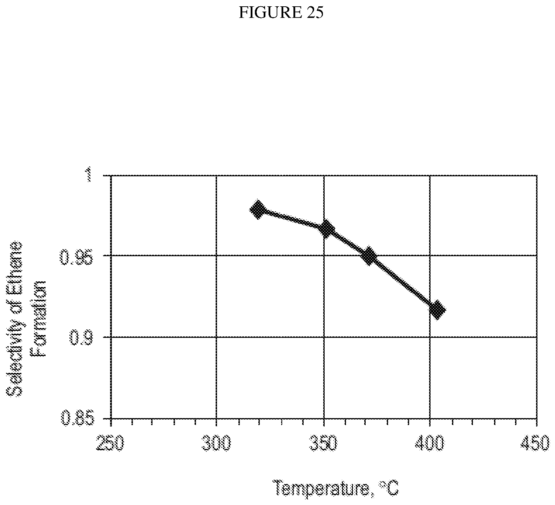

Figure 24 is a plot of the conversion of ethylene formation for sample 9E. Figure 25 is a plot of the selectivity of ethylene formation for sample 9E. The test was run on 0.129 g of catalyst using a gas mixture of 75 vol. % ethane and 25 vol. % oxygen at a flow rate of 600 $cm^3$/h and a GHSV = 3000 $h^{-1}$.

Comparison of these data with results obtained earlier for the sample prepared from oxide (sample 3E) demonstrates a negligible difference in the activity but some loss of the selectivity. The values of T25% ethane conversion are practically identical (365°C and 367°C, respectively), but the difference in the corresponding S values is measurable (96.5% for sample 3E vs. 95% for sample 9E). Some influence of the residual sulfate-ions can be supposed.

Other implementations are also within the scope of the following claims.

**Claims**

1.  A method for preparing a catalyst comprising:

    forming a slurry comprising oxides of molybdenum, tantalum oxide, and tellurium;
    adding $VOSO_4$ to the slurry;
    adding citric acid, oxalic acid, and ethylene glycol to the slurry;
    transferring the slurry to an autoclave;
    heating the autoclave to form a catalyst precursor;
    isolating the catalyst precursor formed in the autoclave; and
    calcining the catalyst precursor to form the catalyst.

2.  The method of claim 1, comprising using anhydrous tantalum oxide as the tantalum oxide in the slurry.

3.  The method of claim 1, comprising preparing the tantalum oxide using $TaCl_5$ by hydrolyzing $TaCl_5$ in ammonium hydroxide to prepare $Ta_2O_5 \cdot 3H_2O$.

4.  The method of claim 1, comprising heating the autoclave at 190 °C for 48 hours.

5.  The method of claim 1, comprising calcining the catalyst precursor by:

    placing the catalyst precursor in a furnace under a purified nitrogen atmosphere;
    ramping a temperature of the furnace from ambient to a temperature of 600°C over 6 hours; and
    holding the temperature at 600°C for 2 hours.

6.  The method of claim 1, wherein the catalyst comprises the formula:

    $$Mo_aV_bTe_cTa_dO_x$$

    wherein:

a is 1.0;
b is about 0.01 to about 0.3;
c is about 0.01 to about 0.09;
d is about 0.01 to about 0.05; and
x is the number of oxygen atoms necessary to render the catalyst electronically neutral.

7. The method of claim 6, wherein:

b is about 0.2 to about 0.4;
c is about 0.03 to about 0.07; and
d is about 0.03 to about 0.05.

8. The method of claim 1, wherein the catalyst has the formula:

$$Mo_1V_{0.3}Te_{0.05}Ta_{0.05}O_x$$

9. The method of claim 8, wherein the catalyst has a temperature of 25% ethane conversion of about 367°C and a selectivity at 25% ethane conversion of about 95%.

10. A process for oxidative dehydrogenation of ethane, the process comprising contacting a gaseous feed comprising ethane and oxygen with a catalyst in a reactor to produce an effluent comprising ethylene, wherein the catalyst has the formula:

$$Mo_aV_bTe_cTa_dO_x$$

wherein:

a is 1.0;
b is about 0.01 to about 0.3;
c is about 0.01 to about 0.09;
d is about 0.01 to about 0.05; and
x is the number of oxygen atoms necessary to render the catalyst electronically neutral, wherein the catalyst is prepared by:

forming a slurry comprising oxides of molybdenum, tantalum oxide, and tellurium;
adding VOSO4 to the slurry;
adding citric acid, oxalic acid, and ethylene glycol to the slurry;
transferring the slurry to an autoclave;
heating the autoclave to form a catalyst precursor;
isolating the catalyst precursor formed in the autoclave; and
calcining the catalyst precursor to form the catalyst.

11. The process of claim 10, wherein:

b is about 0.2 to about 0.4;
c is about 0.03 to about 0.07; and
d is about 0.03 to about 0.05.

12. The process of claim 10, wherein the catalyst has the formula:

$$Mo_1V_{0.3}Te_{0.05}Ta_{0.05}O_x$$

13. The process of any of claims 10 to 12. comprising preparing the catalyst using anhydrous $Ta_2O_5$ without further preparation as a precursor.

14. The process of any of claims 10 to 13. comprising preparing the catalyst using $TaCl_5$ by:

hydrolyzing $TaCl_5$ in ammonium hydroxide to prepare $Ta_2O_5 \cdot 3H_2O$; and
using the $Ta_2O_5 \cdot 3H_2O$ to prepare the catalyst.

**15.** The process of any of claims 10 to 14. wherein the catalyst has a temperature of 25% ethane conversion of about 367°C and a selectivity at 25% ethane conversion of about 95%.

**Patentansprüche**

**1.** Verfahren zur Herstellung eines Katalysators, das Folgendes umfasst:

Bilden einer Aufschlämmung, die Oxide von Molybdän, Tantaloxid und Tellur umfasst;
Zugeben von $VOSO_4$ zur Aufschlämmung;
Zugeben von Citronensäure, Oxalsäure und Ethylenglykol zur Aufschlämmung;
Übertragen der Aufschlämmung in einen Autoklaven;
Erhitzen des Autoklaven, um einen Katalysatorvorläufer zu bilden;
Isolieren des im Autoklaven gebildeten Katalysatorvorläufers; und
Calcinieren des Katalysatorvorläufers, um den Katalysator zu bilden.

**2.** Verfahren nach Anspruch 1, welches die Verwendung von wasserfreiem Tantaloxid als Tantaloxid in der Aufschlämmung umfasst.

**3.** Verfahren nach Anspruch 1, welches das Herstellen des Tantaloxids unter Verwendung von $TaCl_5$ durch Hydrolysieren von $TaCl_5$ in Ammoniumhydroxid, um $Ta_2O_5 \cdot 3H_2O$ herzustellen, umfasst.

**4.** Verfahren nach Anspruch 1, welches das Erhitzen des Autoklaven auf 190 °C für 48 Stunden umfasst.

**5.** Verfahren nach Anspruch 1, welches das Calcinieren des Katalysatorvorläufers durch Folgendes umfasst:

Platzieren des Katalysatorvorläufers in einem Ofen unter einer gereinigten Stickstoffatmosphäre;
Erhöhen der Temperatur des Ofens von Umgebungstemperatur auf eine Temperatur von 600 °C über 6 Stunden; und
Halten der Temperatur bei 600 °C für 2 Stunden.

**6.** Verfahren nach Anspruch 1, wobei der Katalysator folgende Formel aufweist:

$$Mo_aV_bTe_cTa_dO_x,$$

worin:

a = 1,0 ist;
b etwa 0,01 bis etwa 0,3 ist;
c etwa 0,01 bis etwa 0,09 ist;
d etwa 0,01 bis etwa 0,05 ist; und
x die Anzahl der Sauerstoffatome ist, die notwendig sind, um den Katalysator elektronisch neutral zu machen.

**7.** Verfahren nach Anspruch 6, worin:

b etwa 0,2 bis etwa 0,4 ist;
c etwa 0,03 bis etwa 0,07 ist; und
d etwa 0,03 bis etwa 0,05 ist.

**8.** Verfahren nach Anspruch 1, wobei der Katalysator folgende Formel aufweist:

$$Mo_1V_{0,3}Te_{0,05}Ta_{0,05}O_x.$$

**9.** Verfahren nach Anspruch 8, wobei der Katalysator eine Temperatur für 25 % Ethanumwandlung von etwa 367 °C und eine Selektivität bei 25 % Ethanumwandlung von etwa 95 % aufweist.

10. Verfahren zur oxidativen Dehydrierung von Ethan, wobei das Verfahren das In-Kontakt-Bringen eines Gaszufuhrstroms, der Ethan und Sauerstoff umfasst, mit einem Katalysator in einem Reaktor umfasst, um ein Austrittsgas herzustellen, das Ethylen umfasst, wobei der Katalysator folgende Formel aufweist:

$$Mo_aV_bTe_cTa_dO_x,$$

worin:

a = 1,0 ist;
b etwa 0,01 bis etwa 0,3 ist;
c etwa 0,01 bis etwa 0,09 ist;
d etwa 0,01 bis etwa 0,05 ist; und
x die Anzahl der Sauerstoffatome ist, die notwendig sind, um den Katalysator elektronisch neutral zu machen, wobei der Katalysator durch Folgendes hergestellt wird:

Bilden einer Aufschlämmung, die Oxide von Molybdän, Tantaloxid und Tellur umfasst;
Zugeben von $VOSO_4$ zur Aufschlämmung;
Zugeben von Citronensäure, Oxalsäure und Ethylenglykol zur Aufschlämmung;
Übertragen der Aufschlämmung in einen Autoklaven;
Erhitzen des Autoklaven, um einen Katalysatorvorläufer zu bilden;
Isolieren des im Autoklaven gebildeten Katalysatorvorläufers; und
Calcinieren des Katalysatorvorläufers, um den Katalysator zu bilden.

11. Verfahren nach Anspruch 10, worin:

b etwa 0,2 bis etwa 0,4 ist;
c etwa 0,03 bis etwa 0,07 ist; und
d etwa 0,03 bis etwa 0,05 ist.

12. Verfahren nach Anspruch 10, wobei der Katalysator folgende Formel aufweist:

$$Mo_1V_{0,3}Te_{0,05}Ta_{0,05}O_x.$$

13. Verfahren nach einem der Ansprüche 10 bis 12, welches das Herstellen des Katalysators unter Verwendung von wasserfreiem $Ta_2O_5$ ohne weitere Herstellung als Vorläufer umfasst.

14. Verfahren nach einem der Ansprüche 10 bis 13, welches das Herstellen des Katalysators unter Verwendung von $TaCl_5$ durch Folgendes umfasst:

Hydrolysieren von $TaCl_5$ in Ammoniumhydroxid, um $Ta_2O_5 \cdot 3H_2O$ herzustellen; und
Verwenden des $Ta_2O_5 \cdot 3H_2O$, um den Katalysator herzustellen.

15. Verfahren nach einem der Ansprüche 10 bis 14, wobei der Katalysator eine Temperatur für 25 % Ethanumwandlung von etwa 367 °C und eine Selektivität bei 25 % Ethanumwandlung von etwa 95 % aufweist.

**Revendications**

1. Procédé de préparation d'un catalyseur, comprenant les étapes consistant à :

former une suspension constituée d'oxydes de molybdène, d'oxyde de tantale et de tellure ;
ajouter du $VOSO_4$ à la suspension ;
ajouter de l'acide citrique, de l'acide oxalique et de l'éthylène glycol à la suspension ;
transférer la suspension dans un autoclave ;
chauffer l'autoclave pour former un précurseur de catalyseur ;

isoler le précurseur de catalyseur formé dans l'autoclave ; et
calciner le précurseur de catalyseur pour former le catalyseur.

2. Procédé selon la revendication 1, comprenant l'utilisation d'oxyde de tantale anhydre en tant qu'oxyde de tantale dans la suspension.

3. Procédé selon la revendication 1, comprenant la préparation de l'oxyde de tantale en utilisant du $TaCl_5$ par hydrolyse de $TaCl_5$ dans de l'hydroxyde d'ammonium pour préparer du $Ta_2O_5.3H_2O$.

4. Procédé selon la revendication 1, comprenant le chauffage de l'autoclave à 190° pendant 48 heures.

5. Procédé selon la revendication 1, comprenant la calcination du précurseur de catalyseur par l'intermédiaire des étapes consistant à :

placer le précurseur de catalyseur dans un four sous une atmosphère d'azote purifié ;
faire passer la température du four de la température ambiante à une température de 600°C sur 6 heures ; et
maintenir la température à 600°C pendant 2 heures.

6. Procédé selon la revendication 1, dans lequel la catalyseur comprend la formule :

$$Mo_aV_bTe_cTa_dO_x$$

dans laquelle :

a est égal à 1,0 ;
b est d'environ 0,01 à environ 0,3 ;
c est d'environ 0,01 à environ 0,09 ;
d est d'environ 0,01 à environ 0,05 ; et
x est le nombre d'atomes d'oxygène nécessaire pour rendre le catalyseur électroniquement neutre.

7. Procédé selon la revendication 6, dans lequel :

b est d'environ 0,2 à environ 0,4 ;
c est d'environ 0,03 à environ 0,07 ; et
d est d'environ 0,03 à environ 0,05.

8. Procédé selon la revendication 1, dans lequel la catalyseur présente la formule :

$$Mo_1V_{0,3}Te_{0,05}Ta_{0,05}O_x$$

9. Procédé selon la revendication 8, dans lequel le catalyseur présente une température de conversion d'éthane à 25 % d'environ 36°C et une sélectivité de conversion d'éthane à 25 % d'environ 95 %.

10. Procédé de déshydrogénation oxydative d'éthane, le procédé comprenant la mise en contact d'une charge gazeuse comprenant de l'éthane et de l'oxygène avec un catalyseur dans un réacteur pour produire un effluent comprenant de l'éthylène, dans lequel le catalyseur présente la formule :

$$Mo_aV_bTe_cT_{ad}O_x$$

dans laquelle :

a est égal à 1,0 ;
b est d'environ 0,01 à environ 0,3 ;
c est d'environ 0,01 à environ 0,09 ;
d est d'environ 0,01 à environ 0,05 ; et
x est le nombre d'atomes d'oxygène nécessaire pour rendre le catalyseur électroniquement neutre, dans lequel le catalyseur est préparé par l'intermédiaire des étapes consistant à :

former une suspension constituée d'oxydes de molybdène, d'oxyde de tantale et de tellure ;
ajouter du $VOSO_4$ à la suspension ;
ajouter de l'acide citrique, de l'acide oxalique et de l'éthylène glycol à la suspension ;
transférer la suspension dans un autoclave ;
chauffer l'autoclave pour former un précurseur de catalyseur ;
isoler le précurseur de catalyseur formé dans l'autoclave ; et
calciner le précurseur de catalyseur pour former le catalyseur.

**11.** Procédé selon la revendication 10, dans lequel :

b est d'environ 0,2 à environ 0,4 ;
c est d'environ 0,03 à environ 0,07 ; et
d est d'environ 0,03 à environ 0,05.

**12.** Procédé selon la revendication 10, dans lequel le composé présente la formule :

$$Mo_1V_{0,3}Te_{0,05}Ta_{0,05}O_x$$

**13.** Procédé selon l'une quelconque des revendications 10 à 12, comprenant la préparation du catalyseur en utilisant du $Ta_2O_5$ anhydre sans autre préparation en tant que précurseur.

**14.** Procédé selon l'une quelconque des revendications 10 à 13, comprenant la préparation du catalyseur en utilisant du $TaCl_5$ en :

hydrolysant du $TaCl_5$ dans de l'hydroxyde d'ammonium pour préparer du $Ta_2O_5.3H_2O$ ; et
utilisant le $Ta_2O_5.3H_2O$ pour préparer le catalyseur.

**15.** Procédé selon l'une quelconque des revendications 10 à 14, dans lequel le catalyseur présente une température de conversion d'éthane à 25 % d'environ 36°C et une sélectivité de conversion d'éthane à 25 % d'environ 95 %.

FIGURE 1

FIGURE 2A

FIGURE 2B

FIGURE 3

FIGURE 4

FIGURE 5

FIGURE 6

FIGURE 7

FIGURE 8

FIGURE 9

FIGURE 10

FIGURE 11

FIGURE 12

FIGURE 13

FIGURE 14

FIGURE 15

FIGURE 16

FIGURE 17

FIGURE 18

FIGURE 19

FIGURE 20

FIGURE 21

FIGURE 22

FIGURE 23

FIGURE 24

FIGURE 25

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006130288 A

**Non-patent literature cited in the description**

- *CHEMICAL ABSTRACTS*, 7721-01-9
- *CHEMICAL ABSTRACTS*, 1314-61-0
- *CHEMICAL ABSTRACTS*, 1313-27-5
- *CHEMICAL ABSTRACTS*, 1314-62-1
- *CHEMICAL ABSTRACTS*, 7446-07-3
- *CHEMICAL ABSTRACTS*, 77-92-9
- *CHEMICAL ABSTRACTS*, 144-62-7